# EUROPÄISCHE PATENTSCHRIFT

(11) **EP 1 244 640 B1**
(45) Veröffentlichungstag und Bekanntmachung des Hinweises auf die Patenterteilung: **09.02.2005**
(21) Anmeldenummer: 00981308.0
(22) Anmeldetag: 27.11.2000
(51) Int. Cl.: C07D 263/10, C07D 295/22, A61P 7/02, A61K 31/422

(54) **OXAZOLIDINON-DERIVATE**
OXAZOLIDINONE DERIVATIVES
DERIVES D'OXAZOLIDINONE

(30) Priorität: 03.12.1999 DE 19958153
(43) Veröffentlichungstag der Anmeldung: 02.10.2002
(73) Patentinhaber: MERCK PATENT GmbH, 64293 Darmstadt (DE); Yamanouchi Pharmaceutical Company Ltd., Tokyo 174-8612 (JP)
(72) Erfinder: DASENBROCK, Johannes, 64287 Darmstadt (DE); TSAKLAKIDIS, Christos, 69469 Weinheim (DE); WURZIGER, Hanns, 6291 Darmstadt (DE); BARNES, Christopher, 65812 Bad Soden (DE); BÜHRING, Karl-Ulrich, 85567 Grafing (DE); GOODMAN, Simon, 64347 Griesheim (DE)
(86) Internationale Anmeldenummer: PCT/EP2000/011777
(87) Internationale Veröffentlichungsnummer: WO 2001/040201

(56) Entgegenhaltungen:
- EP-A- 0 623 615
- EP-A- 0 697 408
- WO-A-97/10223
- J GANTE ET AL: "A new class of peptidomimetic adhesion receptor antagonists" PEPTIDES,US,ELMSFORD, 1996, Seiten 401-402, XP002103811 ISSN: 0196-9781
- L H PATTERSON: "Rationale for the use of aliphatic N-oxides" CANCER AND METASTASIS REVIEWS,NL,KLUWER ACADEMIC PUBLISHERS, DORDRECHT, Bd. 12, Nr. 2, 1993, Seiten 119-134, XP002118524 ISSN: 0167-7659

## Beschreibung

Die Erfindung betrifft Verbindungen der Formel I 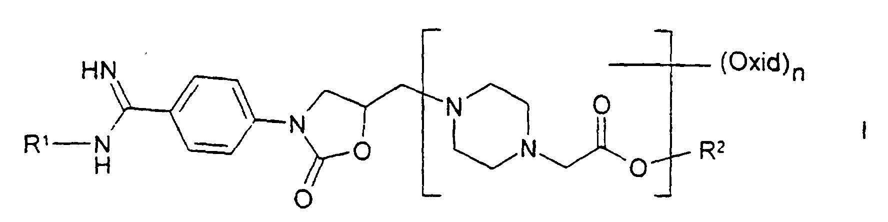 worin
- R¹: H, Ar-CO-, A-CO-, OH, OA, Ar-O-CO-, AO-CO-, Ar-SO₂ oder A-SO₂,
- R²: H, A' oder Benzyl,
- A, A', A": jeweils unabhängig voneinander Alkyl mit 1-6 C-Atomen,
- Ar: unsubstituiertes oder ein-, zwei- oder dreifach durch A, F, Cl, Br, OA", COOA", COOH, CF₃, OH, NO₂, CN, NH₂, NHA, NA₂ oder -O-CO-A" substituiertes Phenyl oder Naphthyl und
- n: 1 oder 2 bedeuten,
sowie deren physiologisch unbedenklichen Salze und Solvate, wobei Verbindungen wird einen C=O Gruppe im Piperazinring ausgenommen sind.

Ähnliche Verbindungen sind z. B. aus EP 0741133 und EP 0623615 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen.
Verbindungen der Formel f, die die Wechselwirkung von Integrinrezeptoren und Liganden, wie z. B. von Fibrinogen an den Fibrinogenrezeptor (Glycoprotein IIb/IIIa) blockieren, verhindern als GPIIb/IIIa-Antagonisten die Ausbreitung von Tumorzellen durch Metastase. Dies wird durch folgende Beobachtungen belegt:
Die Verbreitung von Tumorzellen von einem lokalen Tumor in das vaskuläre System erfolgt durch die Bildung von Mikroaggregaten (Mikrothromben) durch Wechselwirkung der Tumorzellen mit Blutplättchen. Die Tumorzellen sind durch den Schutz im Mikroaggregat abgeschirmt und werden von den Zellen des Immunsystems nicht erkannt.
Die Mikroaggregate können sich an Gefäßwandungen festsetzen, wodurch ein weiteres Eindringen von Tumorzellen in das Gewebe erleichtert wird. Da die Bildung der Mikrothromben durch Fibrinogenbindung an die Fibrinogenrezeptoren auf aktivierten Blutplättchen vermittelt wird, können die GPIIa/IIIb-Antagonisten als wirksame Metastase-Hemmer angesehen werden.

Verbindungen der Formel I hemmen neben der Bindung von Fibrinogen, Fibronectin und des Willebrand-Faktors an den Fibrinogenrezeptor der Blutplättchen auch die Bindung weiterer adhäsiver Proteine, wie Vitronectin, Kollagen und Laminin, an die entsprechenden Rezeptoren auf der Oberfläche verschiedener Zelltypen. Sie verhindern insbesondere die Entstehung von Blutplattchenthromben und können daher zur Behandlung von Thrombosen, Osteoporose, und osteolytischen Erkrankungen eingesetzt werden.
Ferner haben die Verbindungen einen Effekt auf Tumorzellen , indem sie deren Metastasierung hemmen. Somit können sie auch als Anti-Tumor-Mittel eingesetzt werden.
Die Verbindungen eignen sich zudem als antimikrobielle Wirkstoffe, die Infektionen, wie sie beispielsweise durch Bakterien, Pilze oder hefen ausgelöst werden, verhindern können. Die Substanzen können daher vorzugsweise als begleitende antimikrobielle Wirkstoffe gegeben werden, wenn Eingriffe an Organismen vorgenommen werden. bei denen körperfremde Stoffe, wie z.B. Biomaterialien, Implantate, Katheter oder herzschrittmacher, eingesetzt werden. Sie wirken als Antiseptika. Die Wirksamkeit der antimikrobiellen Aktivität kann durch das von P.Valentin-Weigund et al., in Infection and Immunity, 2851-2855 (1988) beschriebene Verfahren nachgewiesen werden.

Die Eigenschaften der Verbindungen können auch nach Methoden nachgewiesen werden, die in der EP-A1-0 462 960 beschrieben sind. Die Hemmung der Fibrinogenbindung an den Fibrinogenrezeptor kann nach der Methode nachgewiesen werden, die in der EP-A1-0 381 033 angegeben ist.

Die thrombozytenaggregationshemmende Wirkung läßt sich in vitro nach der Methode von Born (Nature 4832, 927-929, 1962) nachweisen.

Die Verbindungen der Formel I können als Arzneimittelwirkstoffe in der Human- und Veterinärmedizin eingesetzt werden, zur Bekämpfung von Thrombosen, Osteoporose, Tumorerkrankungen. Apoplexie, Herzinfarkt, Ischämie, Entzündungen, Arteriosklerose und osteolytischen Erkrankungen.

Gegenstand der Erfindung ist ferner ein Verfahren zur Herstellung von Verbindungen der Formel I nach Anspruch 1 sowie ihrer Salze und Solvate, dadurch gekennzeichnet. daß man
eine Verbindung der Formel II 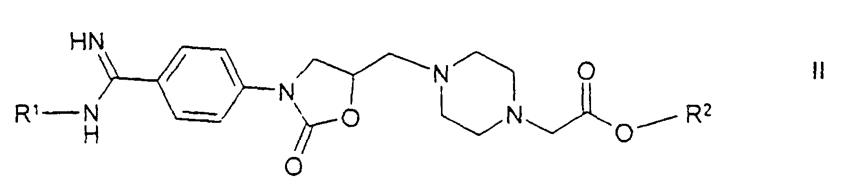 oxidiert,
und/oder
eine basische oder saure Verbindung der Formel I durch Behandeln mit einer Säure oder Base in eines ihrere Salze überführt.

Die Verbindungen der Formel I besitzen mindestens ein chirales Zentrum und können daher in mehreren stereoisomeren Formen auftreten. Alle diese Formen (z. B. D- und L-Formen) und deren Gemische (z. B. die DL-Formen) sind in der Formel I eingeschlossen.

Für die gesamte Erfindung gift, daß sämtliche Reste, die mehrfach auftreten, wie z.B. A und A', gleich oder verschieden sein können, d.h. unabhängig voneinander sind.

In den vor- und nachstehenden Formeln hat Alkyl 1, 2, 3, 4, 5 oder 6 C-Atome und steht vorzugsweise für Methyl, Ethyl, Propyl, Isopropyl, Butyl, Isobutyl, sek.-Butyl oder tert.-Butyl, ferner auch für Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2-, 1,2,2-Trimethylpropyl.

A-CO- bedeutet vorzugsweise Formyl, Acetyl, Propionyl, Butyryl, Pentanoyl oder Hexanoyl.

OA bedeutet vorzugsweise Methoxy, Ethoxy, ferner z.B. Propoxy oder Butoxy.

Bevorzugte Substituenten für Ar sind z.B. A und/oder Hal, OA, NHA, NAA', CN, NO₂, insbesondere z.B. F, Cl, Hydroxy, Methoxy, Ethoxy, Amino, Dimethylamino.

Ar-CO ist Aroyl und bedeutet vorzugsweise Benzoyl oder Naphthoyl.

Ar ist unsubstituiertes, vorzugsweise - wie angegeben - monosubstituiertes Phenyl. im einzelnen bevorzugt Phenyl. o-. m- oder p-Tolyl. o-, m- oder p-Ethylphenyl, o-, m- oder p-Propylphenyl, o-, m- oder p-Isopropylphenyl, o-, m- oder p-tert.-Butylphenyl, o-, m- oder p-Cyanphenyl, o-, m- oder p-Methoxyphenyl, o-, m- oder p-Ethoxyphenyl, o-, m- oder p-Fluorphenyl, o-, m- oder p-Bromphenyl, o-, m- oder p- Chlorphenyl, o-, m- oder p-Aminophenyl, o-, m- oder p-Methylaminophenyl, o-, m- oder p-Dimethylaminophenyl, o-, m- oder p-Nitrophenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2-Chlor-3-methyl-, 2-Chlor-4-methyl-, 2-Chlor-5-methyl-, 2-Chlor-6-methyl-, 2-Methyl-3-chlor-, 2-Methyl-4-chlor-, 2-Methyl-5-chlor-, 2-Methyl-6-chlor-, 3-Chlor-4-methyl-, 3-Chlor-5-methyl- oder 3-Methyl-4-chlorphenyl, 2-Brom-3-methyl-, 2-Brom-4-methyl-, 2-Brom-5-methyl-, 2-Brom-6-methyl-, 2-Methyl-3-brom-, 2-Methyl-4-brom-, 2-Methyl-5-brom-, 2-Methyl-6-brom-, 3-Brom-4-methyl-, 3-Brom-5-methyl- oder 3-Methyl-4-bromphenyl, 2,4- oder 2,5-Dinitrophenyl, 2,5- oder 3,4-Dimethoxyphenyl, 2,3,4-, 2,3,5-, 2,3,6-, 2,4,6- oder 3,4,5-Trichlorphenyl, 2,4,6-Tritert.-Butylphenyl, 2,5-Dimethylphenyl, p-lodphenyl, 4-Fluor-3-chlorphenyl, 4-Fluor-3,5-dimethylphenyl, 2-Fluor-4-bromphenyl, 2,5-Difluor-4-bromphenyl, 2,4-Dichlor-5-methylphenyl, 3-Brom-6-methoxyphenyl, 3-Chlor-6-methoxyphenyl, 2-Methoxy-5-methylphenyl, 2,4,6-Triisopropylphenyl oder Naphthyl.

R² bedeutet vorzugsweise z.B. H, Methyl, Ethyl oder Benzyl, ganz besonders bevorzugt H.

In der Verbindung der Formel I ist der in eckigen Klammern markierte Molekülteil durch Sauerstoff ein- oder zweifach substituiert.
Unter Solvaten versteht man neben den Hydraten z.B. auch Alkoholate wie z.B. Methanolat, Ethanolat, Isopropanolat oder die Addition eines anderen Lösungsmittels.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Ik ausgedrückt werden, die der Formel I entsprechen und worin die nicht näher bezeichneten Reste die bei der Formel I angegebene Bedeutung haben, worin jedoch
- in Ia): n 1 bedeutet;
- in Ib): R¹ H bedeutet;
- in Ic): R² H bedeutet;
- in Id): R¹ H,
n 1 bedeutet;
- in Ie): R¹ H oder AO-CO bedeutet;
- in If): R¹ H oder AO-CO,
R² H und
n 1 bedeutet;
- in Ig): R¹ H, A-CO- oder AO-CO;
R² H und
n 1 bedeutet;
- in Ih): R¹ H, A-CO- oder AO-CO;
R² H oder A' und
n 1 bedeutet;
- in li): R¹ H, A-CO- oder AO-CO;
R² H, A' oder Benzyl,
A, A' jeweils unabhängig voneinander Alkyl mit 1, 2, 3 oder 4 C-Atomen und
n 1 bedeutet;
- in lk): R¹ H oder AO-CO;
R² H, A' oder Benzyl,
A, A' jeweils unabhängig voneinander Alkyl mit 1, 2, 3 oder 4 C-Atomen und
n 1 bedeutet
sowie deren physiologisch unbedenklichen Salze und Solvate.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag. Stuttgart;) beschrieben sind. und zwar unter Reaktionsbedingungen. die für die genannten Umsetzungen bekannt und geeignet sind. Dabei kann man auch von an sich bekannten. hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Verbindungen der Formel I können vorzugsweise erhalten werden, indem man die nicht-oxidierten Vorstufen oxidiert.

Die Oxidation gelingt durch Behandlung mit z.B. einer Persäure, wie z.B. mit Metachlorperbenzoesäure (mCPBA).
Die Anwesenheit eines zusätzlichen inerten Lösungsmittels ist möglich, aber nicht immer erforderlich. Als inerte Lösungsmittel eignen sich vorzugsweise Wasser oder organische Lösungsmittel, die oxidationsunempfindlich sind. Die Reaktionstemperaturen für die Oxidation liegen zweckmäßig zwischen etwa 0 und etwa 50°, vorzugsweise arbeitet man zwischen 15 und 30° (Raumtemperatur).

Die Herstellung der Vorstufen ist in EP 0741133 auf Seite 13, Zeile 47 bis Seite 16, Zeile 34 und in EP 0623615 auf Seite 5, Zeile 19 bis Seite 7, Zeile 32 beschrieben.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage. die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure. Essigsäure. Propionsäure. Pivalinsäure. Diethylessigsäure.

Malonsäure, Bernsteinsäure, Pimelinsäure. Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzoisulfonsäure, p-Toluolsulfonsäure, Naphthalin-mono- und Disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Andererseits kann eine Säure der Formel I durch Umsetzung mit einer Base in eines ihrer physiologisch unbedenklichen Metall- oder Ammoniumsalze übergeführt werden. Als Salze kommen dabei insbesondere die Natrium-. Kalium-, Magnesium-, Calcium- und Ammoniumsalze in Betracht, ferner substituierte Ammoniumsalze, z. B. die Dimethyl-, Diethyloder Diisopropyl-ammoniumsalze, Monoethanol-, Diethanol- oder Diisopropylammoniumsalze, Cyclohexyl-, Dicyclohexylammoniumsalze, Dibenzylethylendiammoniumsalze, weiterhin z. B. Salze mit Arginin oder Lysin.

Die Verbindungen der Formel I enthalten ein oder mehrere chirale Zentren und können daher in racemischer oder in optisch-aktiver Form vorliegen. Erhaltene Racemate können nach an sich bekannten Methoden mechanisch oder chemisch in die Enantiomeren getrennt werden. Vorzugsweise werden aus dem racemischen Gemisch durch Umsetzung mit einem optisch aktiven Trennmittel Diastereomere gebildet. Als Trennmittel eignen sich z.B. optisch aktive Säuren, wie die D- und L-Formen von Weinsäure. Diacetylweinsäure, Dibenzoylweinsäure, Mandelsäure, Äpfelsäure, Milchsäure oder die verschiedenen optisch aktiven Camphersulfonsäuren wie β-Camphersulfonsäure. Vorteilhaft ist auch eine Enantiomerentrennung mit Hilfe einer mit einem optisch aktiven Trennmittel (z.B. Dinitrobenzoylphenylglycin) gefüllten Säule; als Laufmittel eignet sich z.B. ein Gemisch Hexan/Isopropanol/Acetonitril, z.B. im Volumenverhältnis 82:15:3.

Natürlich ist es auch möglich, optisch aktive Verbindungen der Formel I nach den oben beschriebenen Methoden zu erhalten, indem man Ausgangsstoffe verwendet, die bereits optisch aktiv sind.

Gegenstand der Erfindung ist ferner die Verwendung der Verbindungen der Formel I und/oder ihrer physiologisch unbedenklichen Salze zur Herstellung pharmazeutischer Zubereitungen, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale, topische Applikation oder für eine Applikation in Form eines Inhalation-Sprays eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen. zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden, sowie ferner für Implantate.
Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder mehrere weitere Wirkstoffe enthalten, z. B. ein oder mehrere Vitamine.
Für die Applikation als Inhalationsspray können Sprays verwendet werden, die den Wirkstoff entweder gelöst oder suspendiert in einem Treibgas oder Treibgasgemisch (z. B. CO₂ oder Fluorchlorkohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z. B. Ethanol. Inhalationslösungen können mit Hilfe üblicher Inhalatoren verabreicht werden.

Dabei können die erfindungsgemäßen Substanzen in der Regel in Analogie zu anderen bekannten, im Handel befindlichen Verbindungen, insbesondere aber in Analogie zu den in EP 0459256 beschriebenen Verbindungen verabreicht werden, vorzugsweise in Dosierungen zwischen etwa 0,05 und 500 mg, insbesondere zwischen 0,5 und 100 mg pro Dosierungseinheit verabreicht. Die tägliche Dosierung liegt vorzugsweise zwischen etwa 0,01 und 2 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit. Arzneistoffkombination und Schwere der jeweiligen Erkrankung, welcher die Therapie gilt. Die parenterale Applikation ist bevorzugt.

Vor- und nachstehend sind alle Temperaturen in °C angegeben. In den nachfolgenden Beispielen bedeutet "übliche Aufarbeitung": Man gibt, falls erforderlich. Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation.
- Massenspektrometrie (MS):: EI (Elektronenstoß-Ionisation) M⁺
FAB (Fast Atom Bombardment) (M+H)⁺

### Beispiel 1

Zu einer Lösung von 5 g 1-(3-(4-Amidinophenyl)-2-oxo-5Roxazolidinylmethyl)-piperazin-4-essigsäure, die in EP 623615 Beispiel 16 beschrieben ist, in 70 ml Wasser gibt man bei Raumtemperatur 3,7 g mCPBA . Nach 20 Stunden wird wie üblich aufgearbeitet und der Rückstand über HPLC (Wasser/Acetonitril/Essigsäure = 99:0.9:0.1) aufgetrennt.
Man erhält 260 mg 1-(3-(4-Amidinophenyl)-2-oxo-5R-oxazolidsnylmethyl)-piperazin-4-essigsäure-Monooxid.
Die Reaktionsmischung enthält neben nicht-oxidiertem Ausgangsmaterial noch 1-(3-(4-Amidinophenyl)-2-oxo-5R-oxazolidinylmethyl)-piperazin-4-essigsäure-Dioxid.

Das Massenspektrum (FIA-ESI-MS = Flow Injection Analysis - Electrospray lonization MS) zeigt eine molekulare Masse von M+H = 377 (Monoxid) bzw. von M = 393 (Dioxid).

Nach He-Ionisierung zeigt das Spektrum Fragmentionen bei m/z = 304 und m/z = 316.

### Beispiel 2

Zu 350 ml einer 10 %igen wässrigen Natriumhydrogencarbonatlösung gibt man 25,0 g 1-(3-(4-Amidinophenyl)-2-oxo-5R-oxazolidinylmethyl)-piperazin-4-essigsäure. Unter Eiskühlung gibt man bei ca. 5° langsam in Portionen 16,3 g 3-Chlorperbenzoesäure zu. Man rührt 30 Minuten nach und filtriert die Lösung Nach weiteren 2 Stunden fällt ein Niederschlag aus. Man rührt weitere 5 Stunden unter Eiskühlung nach, trennt die ausgefallene Substanz ab und wäscht mit kaltem Wasser, Methanol und Diethylether. Man erhält nach Trocknen 12,0 g 1-(3-(4-Amidinophenyl)-2-oxo-5R-oxazolidinylmethyl)-piperazin-4-essigsäure-Monooxid.

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99.5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0.2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

### Beispiel I: Inhalations-Spray

Man löst 14 g Wirkstoff der Formel I in 10 I isotonischer NaCl-Lösung und füllt die Lösung in handelsübliche Sprühgefäße mit Pump-Mechanismus. Die Lösung kann in Mund oder Nase gesprüht werden. Ein Sprühstoß (etwa 0.1 ml) entspricht einer Dosis von etwa 0,14 mg.

## Patentansprüche

1. Verbindungen der Formel I 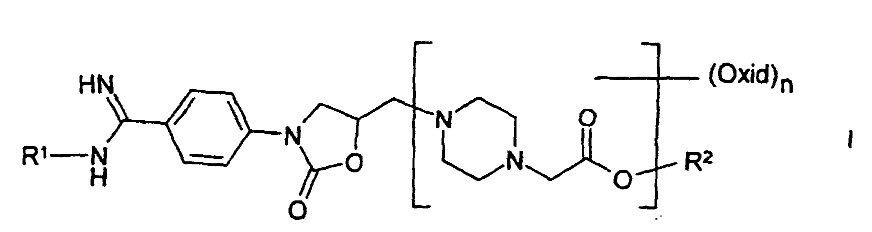 worin
R¹ H, Ar-CO-, A-CO-, OH, OA, Ar-O-CO-, AO-CO-, Ar-SO₂ oder A-SO₂,
R² H, A' oder Benzyl,
A, A', A" jeweils unabhängig voneinander Alkyl mit 1-6 C-Atomen,
Ar unsubstituiertes oder ein-, zwei- oder dreifach durch A, F, Cl, Br, OA", COOA", COOH, CF₃, OH, NO₂, CN, NH₂, NHA, NA₂ oder -O-CO-A" substituiertes Phenyl oder Naphthyl und
n 1 oder 2 bedeuten,
sowie deren physiologisch unbedenklichen Salze und Solvate, wobei Verbindungen mit einer C=O Gruppe im Piperazinring ausgenommen sind.

2. Enantiomere oder Diastereomere der Verbindungen der Formel I gemäß Anspruch 1.

3. Verfahren zur Herstellung von Verbindungen der Formel I nach Anspruch 1 sowie ihrer Salze und Solvate, **dadurch gekennzeichnet, daß** man eine Verbindung der Formel II 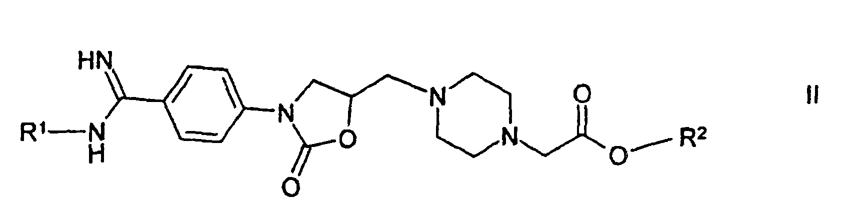 oxidiert,
und/oder
eine basische oder saure Verbindung der Formel I durch Behandeln mit einer Säure oder Base in eines ihrere Salze überführt.

4. Verbindungen der Formel I gemäß der Ansprüche 1 oder 2 sowie ihrer physiologisch unbedenklichen Salze und Solvate als Arzneimittel.

5. Arzneimittel nach Anspruch 4 als selektive GP IIbIIIa Antagonisten.

6. Arzneimittel nach Anspruch 4 oder 5 zur Bekämpfung von Thrombosen, Osteoporose, Tumorerkrankungen, Apoplexie, Herzinfarkt, Ischämie, Entzündungen, Arteriosklerose und osteolytischen Erkrankungen.

7. Pharmazeutische Zubereitung, enthaltend mindestens ein Arzneimittel gemäß einem der Ansprüche 4 bis 6 sowie gegebenenfalls Trägerund/oder Hilfsstoffe und gegebenenfalls andere Wirkstoffe.

8. Verwendung von Verbindungen gemäß der Ansprüche 1, 2 oder 4 und/oder ihre physiologisch unbedenklichen Salze und Solvate zur Herstellung eines Arzneimittels zur Bekämpfung von Thrombosen, Osteoporose, Tumorerkrankungen, Apoplexie, Herzinfarkt, Ischämie, Entzündungen, Arteriosklerose und osteolytischen Erkrankungen.

## Claims

1. Compounds of the formula I 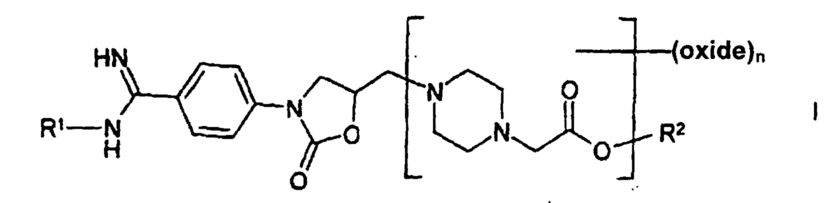 in which
R¹ denotes H, Ar-CO-, A-CO-, OH, OA, Ar-O-CO-, AO-CO-, Ar-SO₂ or A-SO₂,
R² denotes H, A' or benzyl,
A, A', A" each, independently of one another, denote alkyl having 1-6 C atoms,
Ar denotes phenyl or naphthyl, each of which is unsubstituted or mono-, di- or trisubstituted by A, F, Cl, Br, OA", COOA", COOH, CF₃, OH, NO₂, CN, NH₂, NHA, NA₂ or -O-CO-A", and
n denotes 1 or 2,
and physiologically acceptable salts and solvates thereof, where compounds containing a C=O group in the piperazine ring are excluded.

2. Enantiomers or diastereomers of the compounds of the formula I according to Claim 1.

3. Process for the preparation of compounds of the formula I according to Claim 1 and salts and solvates thereof, **characterised in that** a compound of the formula II 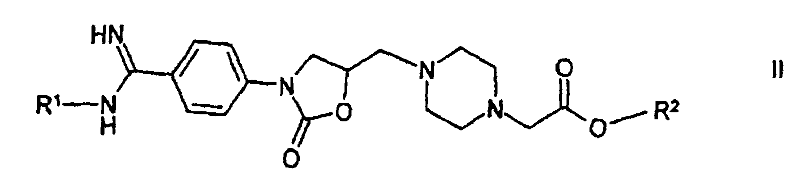 is oxidised,
and/or
a basic or acidic compound of the formula I is converted into one of its salts by treatment with an acid or base.

4. Compounds of the formula I according to Claims 1 or 2 and physiologically acceptable salts and solvates thereof as medicaments.

5. Medicaments according to Claim 4 as selective GP IIbIIIa antagonists.

6. Medicaments according to Claim 4 or 5 for combating thromboses, osteoporosis, tumour diseases, apoplexy, cardiac infarction, ischaemia, inflammations, arteriosclerosis and osteolytic diseases.

7. Pharmaceutical composition comprising at least one medicament according to one of Claims 4 to 6 and optionally excipients and/or adjuvants and optionally other active ingredients.

8. Use of compounds according to Claims 1, 2 or 4 and/or physiologically acceptable salts and solvates thereof for the preparation of a medicament for combating thromboses, osteoporosis, tumour diseases, apoplexy, cardiac infarction, ischaemia, inflammations, arteriosclerosis and osteolytic diseases.

## Revendications

1. Composés de la formule I 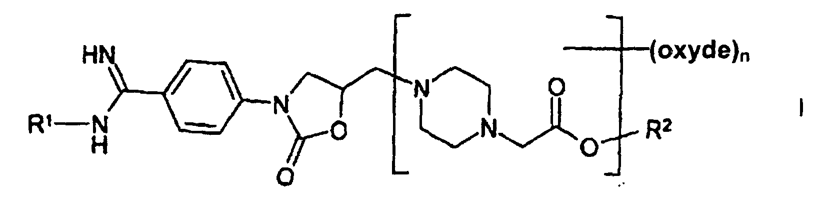 dans laquelle
R¹ représente H, Ar-CO-, A-CO-, OH, OA, Ar-O-CO-, AO-CO-, Ar-SO₂ ou A-SO₂,
R² représente H, A' ou benzyle,
A, A', A" chacun, indépendamment les uns des autres, représente alkyle comportant de 1 à 6 atomes de C,
Ar représente phényle ou naphthyle, dont chacun est non substitué ou mono-, di- ou trisubstitué par A, F, CI, Br, OA", COOA", COOH, CF₃, OH, NO₂, CN, NH₂, NHA, NA₂ ou -O-CO-A", et
n représente 1 ou 2,
et leurs sels et solvates physiologiquement acceptables, où des composés contenant un groupe C=O dans l'anneau pipérazine sont exclus.

2. Enantiomères ou diastéréomères des composés de la formule I selon la revendication 1.

3. Procédé pour la préparation de composés de la formule I selon la revendication 1 et de leurs sels et solvates, **caractérisé en ce que**
un composé de la formule II 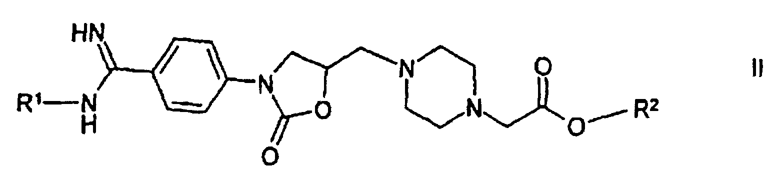 est oxydé,
et/ou
un composé basique ou acide de la formule I est converti selon l'un de ses sels par traitement avec un acide ou une base.

4. Composés de la formule I selon les revendications 1 ou 2 et leurs sels et solvates physiologiquement acceptables en tant que médicaments.

5. Médicaments selon la revendication 4 en tant qu'antagonistes GP IIbIIIa sélectifs.

6. Médicaments selon la revendication 4 ou 5 pour combattre les thromboses, l'ostéoporose, les maladies tumorales, l'apoplexie, l'infarctus cardiaque, l'ischémie, les inflammations, l'artériosclérose et les maladies ostéolytiques.

7. Composition pharmaceutique comprenant au moins un médicament selon l'une des revendications 4 à 6 et en option, des excipients et/ou des adjuvants et en option, d'autres ingrédients actifs.

8. Utilisation de composés selon les revendications 1, 2 ou 4 et/ou de leurs sels et solvates physiologiquement acceptables pour la préparation d'un médicament pour combattre les thromboses, l'ostéoporose, les maladies tumorales, l'apoplexie, l'infarctus cardiaque, l'ischémie, les inflammations, l'artériosclérose et les maladies ostéolytiques.
